# EUROPEAN PATENT SPECIFICATION

(11) **EP 0 450 132 B1**
(45) Date of publication and mention of the grant of the patent: **27.09.1995**
(21) Application number: 90106678.7
(22) Date of filing: 06.04.1990
(51) Int. Cl.: A61M 1/36

(54) **Apparatus for treating fluids in a circuit**
Gerät zur Behandlung von Flüssigkeiten in einem Kreislauf
Appareil pour le traitement de liquides dans un circuit

(43) Date of publication of application: 09.10.1991
(73) Proprietor: Fresenius AG, 61350 Bad Homburg (DE)
(72) Inventor: Jaffrin, Michel, F-60206 Compiègne Cédex (FR); Ding, Luhui, F-60206 Compiègne Cédex (FR)
(74) Representative: Fuchs Mehler Weiss

(56) References cited:
- EP-A- 0 093 542
- TRANSACTIONS OF THE AMERICAN SOCIETY FOR ARTIFICIAL INTERNAL ORGANS, no. 27, 1981, pages 639-643, Washington, DC, US; K. ABEL et al.: "A practical secondary-flow hemodialyzer"
- JOURNAL OF MEMBRANE SCIENCE, no. 44, 1989, pages 115-129, Amsterdam, NL; M.Y. JAFFRIN et al.: "Innovative processes for membrane plasma separation"

## Description

The present invention relates to an apparatus for treating fluids in a circuit comprising a source of fluid to be treated and a receiver for the treated fluid, a fluid circuit comprising at least a first compliant fluid line leading from said source to the inlet of the membrane device, a pump interconnected in said first fluid line being operative to deliver a substantially continuous fluid flow to said membrane device, a second compliant fluid line leading from the outlet of said membrane device to said receiver and at least one device being operative to create a pulsative flow in said fluid circuit.

The apparatus of the invention can be employed in blood treating apparatuses most advantageously as for instance in the field of hemofiltration, plasmapheresis and hemodialysis.

Performance of known devices for treating blood is limited by a combination of factors: Platelet and red cell concentration polarization, membrane plugging by protein absorption, low blood flows especially in plasma collection from donors, and hemolysis.

It has already been suggested (Journal of Membrane Science, 44 (1989) 115-129) to improve performance by generation of microvortices by circulating a pulsatile blood flow over a dimpled membrane. There, the pulsations were produced by intermittent squeezing of a compliant tube by a moving plate. Under pulsations at frequencies ranging from 2 to 4 Hz the filtrate output was increased by 50%.

For similar reasons in EP-B1-93542 it has been suggested to employ a valve downstream of a blood pump. The valve normally is open. It can, however, be closed by a control circuit on request in order to produce pulsations in the fluid line. The valve is said to be able to produce sharp curve patterns of pulsations and therefore its use has advantages over an intermittent rotation of a well known roller pump, widely in use in such devices.

It is an object of the present invention to provide means by which performance of said apparatus is further increased by using the known effect of inducing pulsations into the fluid to be treated.

This object is attained in the present invention by using a single-roller pump as the device to create pulsations in fluid to be treated of the magnitude which cause a temporary positive pressure gradient between the outlet and the inlet of the membrane device.

The single-roller pump can be considered as an active shutting device in contrast to the known passive valve device in the sense, that the single-roller pump not only shuts the fluid line(s) upon rotation by clamping the fluid line(s) but also conveys parts of fluid volumes contained in the fluid lines back to the membrane device. Upon release of the fluid lines by the single-roller pump the built-up pressure in the fluid lines enables a high flow rate. The pulsations produced thereby are as strong as to create a temporary positive transmembrane pressure gradient across the membrane device when the single-roller pump clamps at least one of the fluid lines, resulting in a fluid flow from the outlet to the inlet of the membrane device. Thus, a temporary reverse fluid flow is the result contributing to the cleaning effect in the membrane device and thereby reducing the membrane resistance.

An important factor in the apparatus of the invention is the compliance of the fluid lines. Reasonable results have been achieved with silicone as material for the fluid lines.

Increases of performance of the apparatus of up to 100% have been monitored over conventional devices.

Said single-roller pump may be arranged so as to clamp the fluid line between the outlet of the membrane device and said fluid receiver. A single-roller device refers to a clamping means and a fluid delivery means in combination which means act commonly and in certain intervalls.

Alternatively, the single-roller pump may be arranged to clamp the first fluid line between the fluid pump and the inlet of the membrane device and the second fluid line between the outlet of said membrane device and the fluid receiver, alternately and vice versa.

Within both embodiments magnificent results have been obtained.

The clamping sections of said single-roller pump may be adjustable with regard to the fluid lines to be clamped for adjustments of the built-up pressure in the fluid lines during clamping of said fluid lines.

Embodiments of the apparatus of the present invention will now be described by way of examples with reference to the accompanying drawings, in which:
- Figure 1: is a diagram of a first embodiment of the apparatus according to the invention,
- Figure 2: shows curves of test data measured in an apparatus according to figure 1,
- Figure 3: is a diagram of another embodiment of the apparatus of the invention,
- Figure 4: shows curves of test data obtained from an apparatus according to figure 3.

Hereinafter like parts are designated with like reference numbers.

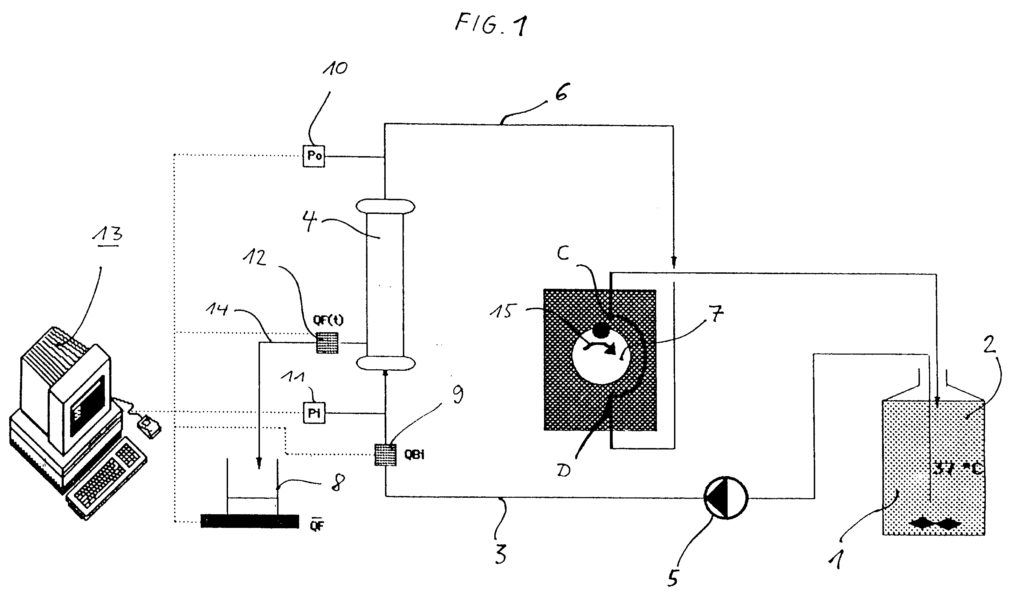

The apparatus shown in figure 1 comprises a fluid source 1 containing the fluid, for instance blood of a patient, to be treated. The fluid is conveyed in a first fluid line 3 by means of a pump 5 interconnected in said first fluid line 3. The fluid line 3 is compliant and is made out of any suitable elastic material, preferably silicone or PVC. The pump 5 is of a type delivering a substantially continuous fluid flow to a membrane device via fluid line 3 and may be a conventional double-roller pump, widely in use in the field of the present invention.

After being delivered to the inlet of the membrane device 4, the fluid passes through the membrane device 4 to its outlet. From the outlet the fluid flows in the second fluid line 6 which is compliant as is said first fluid line 3.

A single-roller pump 7 is arranged such that it acts on said second fluid line 6 between the outlet of the membrane device 4 and a fluid receiver 2.

As indicated by arrow 15 direction of rotation of said single-roller pump 7 is such that upon clamping fluid lines 6 between locations C and D pump 7 counteracts pump 5 in fluid line 3 leading to a rapid building-up of pressure and resulting in a tendency to expand the compliant fluid lines 3 and 6. Due to their compliance fluid lines act like intermediate energy storages which release their stored energy once their single-roller pump 7 releases fluid line 6 and thereby contribute to an acceleration of the fluid flow.

For evaluation of test data during operation of the apparatus an inlet low rate sensor 9 is interconnected in fluid line 3 as is an inlet pressure sensor 11. Filtrate is led from membrane device 4 via fluid line 14 to a collecting container 8. An filtration flow rate sensor 12 is interconnected in fluid line 14 to monitor the filtration flow rate. The outlet pressure of membrane device 4 is monitored by means of outlet pressure sensor 10 located at fluid line 6 adjacent to the outlet of the fluid chamber of the membrane device 4.

All of said sensors 9, 10, 11 and 12 transmit the monitored values to a control unit 13, where the test data are analyzed.

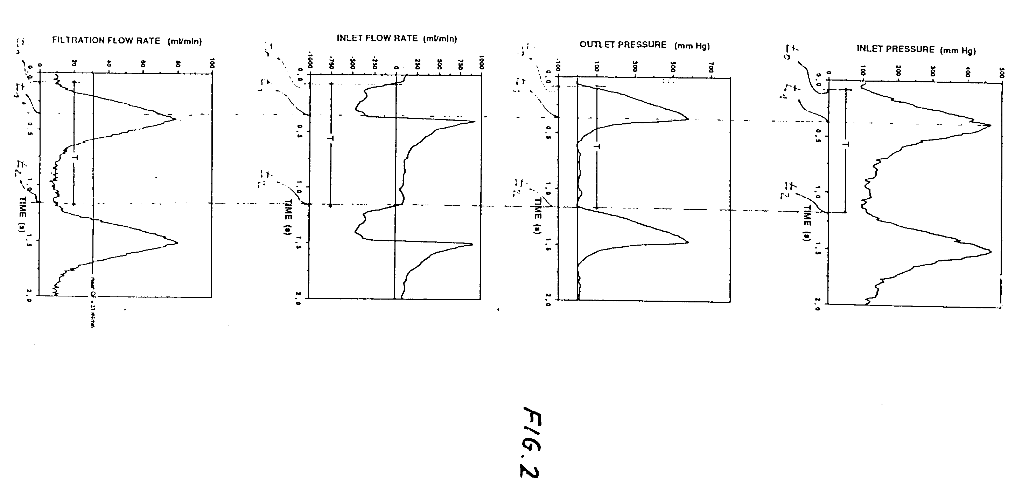

Referring now to figure 2 monitored values will be discussed. There the inlet pressure, outlet pressure, inlet flow rate and filtration flow rate are plotted against time.

At t_{O} single-roller pump 7 engages fluid line 6 at location C and begins clamping it and pushing a certain volume of fluid back to membrane device. As is apparent from the curves, inlet and outlet pressure are subject of an immediate in phase rapid increase. Interestingly, outlet pressure is higher than inlet pressure from this time on until single-roller pump 7 disengages fluid line 6. In consequence a temporary positive pressure gradient is created from the outlet to the inlet of membrane device 4 resulting in a negative inlet flow rate, i.e. fluid is forced back through the inlet against the pressure caused by pump 5. At this state the filtration flow rate commences to increase considerably.

This state will be in effect until single-roller pump 7 releases fluid line 6 at t₁. Then a rapid decrease of inlet and outlet pressure can be observed. Decrease of outlet pressure is steeper than of inlet pressure due to the fact that the compliant fluid line walls under tension now release as well and that pressure caused by pump 5 is present, continuously. In consequence fluid flow will reverse again and find its way from the inlet to the outlet of membrane device 4, as is represented by a positive inlet flow rate roughly from said moment of release of fluid line 6 on. Also the filtration flow rate decreases until a whole period T of single-roller pump 7 is over at t₂ and the process repeats.

As a result a significantly increased mean filtration rate has been observed. It is believed that due to the rapid increases and decreases in flow have the positive effect on cleaning the membrane in membrane device 4.

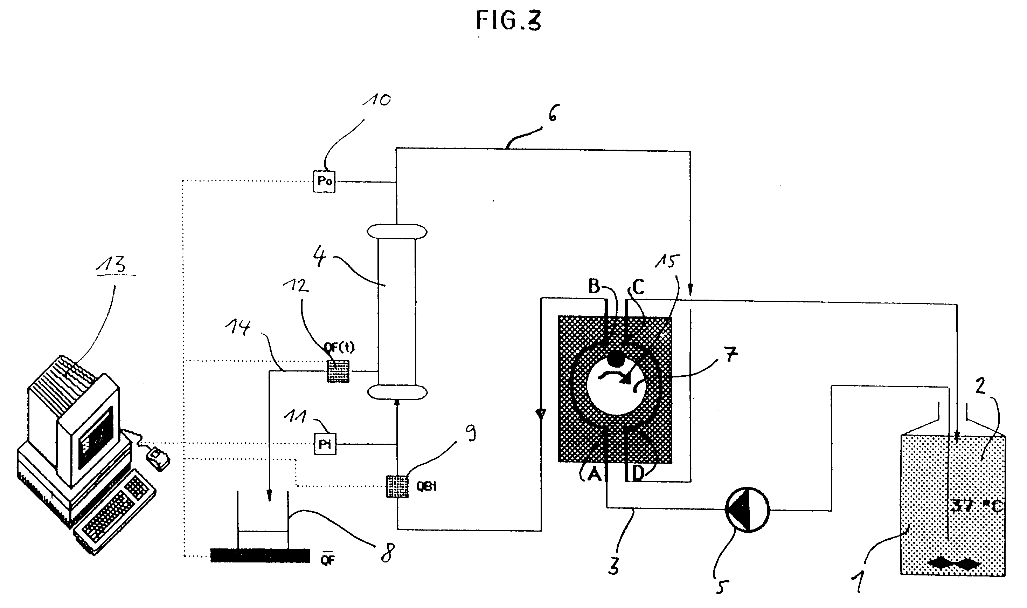

Figure 3 illustrates another embodiment of the apparatus according to the invention. The difference to the apparatus shown in figure 1 is the different arrangement of the single-roller pump 7. According to figure 3 single-roller pump 7 is arranged to alternatively clamp fluid line 3 between the pump 5 and the inlet of membrane device 4 between locations designated A and B and fluid line 6 between the outlet of membrane device 4 and receiver 2 between locations designated C and D. Preferably the single-roller pump 7 clamps in the circle of 360° about 120 - 150° each of the lines 3 or 6, rest 30 - 60° unclamped.

Interestingly, as indicated by arrow 15, direction of rotation of said single-roller pump 7 is such that upon clamping fluid line 6 between locations C and D pump 7 counteracts pump 5 as it does in the predescribed embodiment. However, upon clamping fluid line 3 between locations A and B pump 7 supports pump 5 in conveying the fluid to membrane device 4.

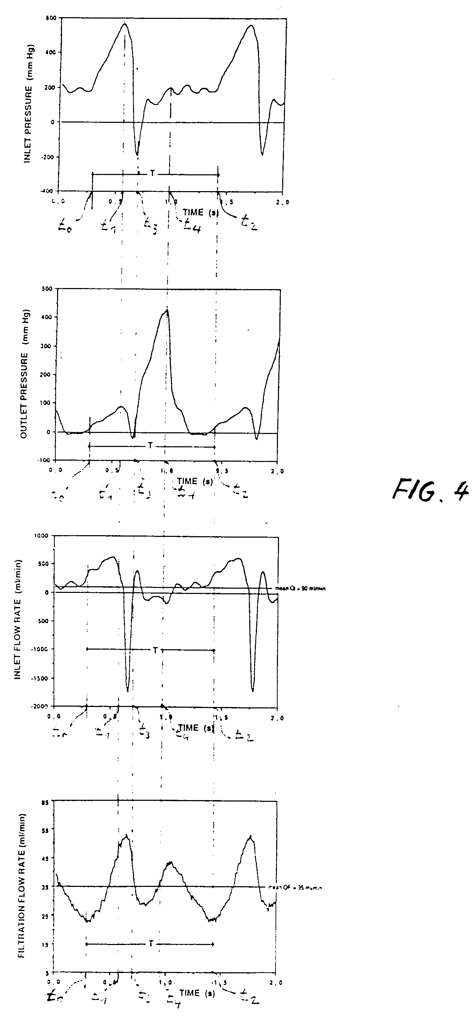

Test results corresponding to the embodiment of figure 3 are plotted in figure 4. What is important for interpretation of the curves to know is that single-roller pump 7 has a higher delivery than pump 5 preferably by the factor 2 or more. .

Considering now the moment at t_{O} single-roller pump 7 commences to clamp fluid line 3 until moment t₁. Since delivery of pump 7 is higher than the one of pump 5 inlet pressure is higher than outlet pressure and a subpressure is created in fluid lines 3 and 6. Upon release of fluid line 3 at t₁ a pressure compensation wave occurs in fluid lines 3 and 6 as well as in membrane device 4 resulting in a rapid decrease of inlet pressure and in a reverse flow at the inlet of the membrane device 4.

At t₃ the single-roller pump 7 engages fluid line 6 at location C. In consequence outlet pressure is subject of an immediate increase. In the present embodiment inlet pressure does not increase as rapid as it does in the first embodiment of the apparatus of the invention due to compensation effects within the fluid circuit. Finally, at t₄ single-roller pump 7 disengages the fluid line 6 at location D.

The mean filtration flow rate in this second embodiment is even slightly higher than in the first embodiment and considerably higher than in known apparatuses.

From the foregoing it becomes apparent that relevant operational parameters of the apparatus of the invention are: difference between deliveries of pump 5 and single-roller pump 7, length of the clamping sections of said single-roller pump 7 with regard to the fluid lines to be clamped and the compliance of the fluid lines 3 and 6.

For the sake of optimizing performance of the apparatus said clamping sections of the single-roller pump may be adjustable which is considered as an essential of the present invention. In addition or independently thereof velocities of pump 5 and 7 may be varied during operation of apparatus.

It is emphazised that the device referred to as single-roller pump may be any suitable means able not only to clamp the fluid line(s) but also to convey a certain volume of the fluid against the main direction of conveyance of the fluid from fluid source 1 to receiver 2. For instance a linear peristaltic pump may be installed for said purpose.

## Claims

1. Apparatus for treating fluids in a circuit comprising a source (1) of fluid to be treated and a receiver (2) for the treated fluid, a fluid circuit comprising at least a first compliant fluid line (3) leading from said source (1) to the inlet of a membrane device (4), a pump (5) interconnected in said first fluid line (3) being operative to deliver a substantially continuous fluid flow to said membrane device (4), a second compliant fluid line (6) leading from the outlet of said membrane device (4) to said receiver (2) and at least one device (7) being operative to create a pulsatile flow in said fluid circuit, characterized in that the device (7) is a clamping and conveying device operative to cause a temporary positive pressure gradient between the outlet and the inlet of the membrane device (4) by conveying fluid contained in the second fluid line (6) back to the outlet of the membrane device (4) during its active engagement period, such that a temporary reverse fluid flow in the membrane device (4) is obtained within said period.

2. Apparatus as claimed in claim 1 with said device (7) being arranged to alternately clamp said first fluid line (3) between the fluid pump (5) and the inlet of the membrane device (4) and said second fluid line (6) between the outlet of said membrane device (4) and the fluid receiver (2).

3. Apparatus as claimed in any proceeding claims wherein the clamping sections of said device (7) with regard to the fluid lines are adjustable.

4. Apparatus as claimed in anyone of the above claims wherein the material of said fluid lines (3, 6) is silicone.

## Patentansprüche

1. Gerät zur Behandlung von Fluiden in einem Kreislauf, aufweisend eine Quelle (1) des zu behandelnden Fluids und ein Aufnahmegefäß (2) für das behandelte Fluid, wobei ein Fluidkreislauf zumindest eine erste nachgebende Fluidleitung (3), die von der Quelle (1) zu dem Einlaß der Membranvorrichtung (4) führt, eine Pumpe (5), die in die erste Fluidleitung (3) eingebaut ist, die derart arbeitet, daß sie einen im wesentlichen kontinuierlichen Fluidfluß zu der Membranvorrichtung (4) überträgt, eine zweite nachgebende Fluidleitung (6), die von dem Auslaß der Membranvorrichtung (4) zu dem Aufnahmegefäß (2) führt und zumindest eine Vorrichtung (7) aufweist, die derart arbeitet, daß sie einen pulsierenden Fluß in dem Fluidkreislauf erzeugt, **dadurch gekennzeichnet**, daß die Vorrichtung (7) eine Abklemm- und Fördervorrichtung ist, die derart arbeitet, daß sie einen temporären, positiven Druckgradienten zwischen dem Auslaß und dem Einlaß der Membranvorrichtung (4) erzeugt, indem das Fluid, das in der zweiten Fluidleitung (6) enthalten ist, während der Zeitspanne des aktiven Eingriffs zurück zum Auslaß der Membranvorrichtung (4) gefördert wird, so daß ein temporärer Rückstrom des Fluids in die Membranvorrichtung in dieser Zeitspanne erzielt wird.

2. Gerät nach Anspruch 1, bei dem die Vorrichtung (7) derart angeordnet ist, daß sie abwechselnd die erste Fluidleitung (3) zwischen der Fluidpumpe (5) und dem Einlaß der Membranvorrichtung (4) und die zweite Fluidleitung (6) zwischen dem Auslaß der Membranvorrichtung (4) und dem Aufrahmegefäß (2) für das Fluid abklemmt.

3. Gerät nach einem der vorhergehenden Ansprüche, in dem die Abklemmbereiche der Vorrichtung (7) in bezug auf die Fluidleitungen justierbar sind.

4. Gerät nach einem der vorhergehenden Ansprüche, bei dem das Material, aus dem die Fluidleitungen (3, 6) bestehen, Silikon ist.

## Revendications

1. Appareil de traitement de fluides dans un circuit comprenant une source (1) de fluide à traiter et un collecteur (2) de fluide traité, un circuit de fluide comprenant au moins un premier conduit (3) de fluide souple raccordant ladite source (1) à l'entrée d'un dispositif (4) à membrane, une pompe (5) interconnectée dans ledit premier conduit (3) de fluide étant opérante pour délivrer un écoulement de fluide sensiblement continu audit dispositif (4) à membrane, un second conduit (6) de fluide souple raccordant la sortie dudit dispositif (4) à membrane audit collecteur (2) et au moins un dispositif (7) étant opérant pour créer un écoulement par impulsions dans ledit circuit de fluide, caractérisé en ce que le dispositif (7) est un dispositif de compression et de transit opérant pour produire un gradient de pression temporaire positif entre la sortie et l'entrée du dispositif (4) à membrane en refoulant le fluide contenu dans le second conduit (6) de fluide vers la sortie du dispositif (4) à membrane pendant sa période active d'engagement, de telle sorte qu'un écoulement temporaire inverse de fluide dans le dispositif (4) à membrane est obtenu dans ladite période.

2. Appareil selon la revendication 1 avec ledit dispositif (7) étant agencé pour comprimer alternativement ledit premier conduit (3) de fluide entre la pompe (5) de fluide et l'entrée du dispositif (4) à membrane et ledit second conduit (6) de fluide entre la sortie dudit dispositif (4) à membrane et le collecteur (2) de fluide.

3. Appareil selon une quelconque des revendications précédentes où les sections de compression dudit dispositif (7) par rapport aux conduits de fluide sont réglables.

4. Appareil selon une quelconque des revendications précédentes où la matière desdits conduits (3, 6) de fluide est le silicone.
